# EUROPÄISCHE PATENTANMELDUNG

(11) **EP 1 801 197 A1**
(43) Veröffentlichungstag der Anmeldung: **27.06.2007**
(21) Anmeldenummer: 06126712.6
(22) Anmeldetag: 20.12.2006
(51) Int. Cl.: C12M 1/107, C12M 1/16, B01D 53/84

(54) **Verfahren zur Verwertung gasförmiger Kohlenstoffquellen und Photobioreaktor**

(30) Priorität: 22.12.2005 DE 102005062726; 22.12.2005 DE 102005062727
(71) Anmelder: Mikrobiologisch-analytisches Labor GmbH, 09366 Stolberg (DE)
(72) Erfinder: Trommler, Klaus, 09387, Jahnsdorf (DE); Weber, Antje, 08280, Aue (DE); Keller, Annett, 08352, Pöhla (DE); Fiedler, Michael, 09123, Chemnitz (DE)
(74) Vertreter: Eisenführ, Speiser & Partner

(57) **Zusammenfassung**

Die Erfindung betrifft ein Verfahren mit der Aufgabe, unter Nutzung beliebiger CO₂-Quellen Kohlendioxid biotechnologisch nahezu vollständig in ein Methan-Sauerstoff-Gemisch bzw. in Methan umzuwandeln, wobei auch Gase mit geringen CO₂-Anteilen, wie beispielsweise Luft, mit einem CO₂ -Gehalt von ca. 0,03 %, entsprechend aufbereitet werden können, sowie einen Photobioreaktor zur Durchführung des Verfahrens. Erfindungsgemäß durchläuft das Input-Gas einen gasdicht geschlossenen Photobioreaktor mittels eines geschlossenen Gaskreislaufes so oft, bis eine nahezu vollständige biotechnologische Umwandlung von C0₂ in ein Methan-Sauerstoff-Gemisch bzw. in Methan erfolgt ist.

## Beschreibung

Die vorliegende Erfindung betrifft ein Verfahren mit der Aufgabe, unter Nutzung beliebiger C0₂-Quellen Kohlendioxid biotechnologisch nahezu vollständig in ein Methan-Sauerstoff-Gemisch bzw. in Methan umzuwandeln, wobei auch Gase mit geringen CO₂-Anteilen, wie beispielsweise Luft mit einem CO₂ -Gehalt von ca. 0,03 %, entsprechend aufbereitet werden können, sowie einen Photobioreaktor zur Durchführung des Verfahrens. Die Erfindung betrifft gleichfalls die zelluläre Verwertung weiterer verstoffwechselbarer Gase, wie z. B. Methan.

Die meisten Verfahren zur Aufzucht bzw. Kultivierung von Mikroalgen haben die Produktion von Biomasse und deren nachfolgende Verwertung zum Ziel.

Verfahren und (Photo-) Bioreaktoren zur Aufzucht bzw. Kultivierung phototropher Mikroorganismen unter Nutzung gasförmiger Nahrungsquellen sind vielfältig bekannt (DD 291092, DE 43 17 006, DE 100 49 437, DE 101 64 458, DE 196 11 855, DE 199 16 597, DE 296 07 285, DE 694 08 129, DE 10 2004 019 234, EP 0 889 118, EP 0 911386, EP 1 498 475).

Dabei wird auch CO₂ aus Abgasen und aus Anaerobreaktoren (Biogas) als Kohlenstoffquelle für die Algenproduktion genannt. Nachteilig ist dabei, dass nicht durch Algen gebundenes Kohlendioxid bzw. in der Dunkelphase der Photosynthese von Algen gebildetes Kohlendioxid freigesetzt wird oder gesondert aufgefangen werden muss bzw. das austretende Gas immer noch erhebliche Anteile an Kohlendioxid enthält.

Allen bisher beschriebenen Verfahren, gleichgültig ob offene oder geschlossene Fermenter verwendet werden, ist gemeinsam, dass das in den Photobiorektor eingeleitete Gas diesen nach Passage der Reaktorflüssigkeit bzw. nach Ablauf der Fermentation in die Umgebung verlassen kann bzw. einer Einrichtung zur Gasverwertung zugeführt wird. Nicht durch Algen gebundenes Kohlendioxid bzw. in der Dunkelphase der Photosynthese von Algen gebildetes Kohlendioxid wird somit freigesetzt oder muss gesondert aufgefangen werden.

Beschrieben wird ebenfalls ein Verfahren zur Verminderung des Anteils an Kohlendioxid in brennbaren Gasen zur Erhöhung des energetischen Wertes des Endgases (DE 197 21 280).

Nachteilig für den Prozess der Abreicherung von Kohlendioxid aus Biogas, wie er in DE 197 21 280 beschrieben ist, ist das Erfordernis von drei Reaktorsystemen (Assimilator, Dissimilator und Photosynthesereaktor), um einer Einrichtung zur Gasverwertung Methan und Sauerstoff zuführen zu können. Nachteilig ist weiterhin, dass das aus dem Assimilator austretende Gas immer noch erhebliche Anteile an Kohlendioxid enthält.

In DE 197 21 243 C2 ist ein Verfahren zur effizienten Nutzung von Biogas zur Erzeugung von Polyhydroxybuttersäure beschrieben.

Bei den oben genannten Verfahren und Photobioreaktoren müssen Nachteile bezüglich der Gasqualität, der Gasverwertung, der Gasausbeute, der Ausbeute an Biomasse und der Qualität der Biomasse in Kauf genommen werden.

Der Erfindung liegt deshalb die Aufgabe zugrunde, die oben genannten Nachteile zu vermeiden und ein Verfahren und einen Photobioreaktor zu entwickeln, mit dem eine maximale CO₂ -Ausnutzung und eine optimale Gasreinigung gewährleistet ist.

Erfindungsgemäß wird die Aufgabe mit einem Verfahren gelöst, in welchem Kohlendioxid rein oder in einem Gasgemisch befindlich einem Photobioreaktor zugeführt wird, der durch einen geschlossenen Gaskreislauf charakterisiert ist und in dem das zugeführte Gas in der Regel mindestens einmal zirkuliert. Die weitere Anzahl der Zirkulationen ist beliebig und einzig zweckbestimmt.

Erfindungsgemäß ist als wesentlicher Verfahrensschritt ein zusätzlicher Gaskreislauf (Kurzschlußkreislauf) enthalten, der direkt zum Photobioreaktor zurückführt, sowie vorzugsweise eine Gasreinigung, die bei Bedarf in diesen Kurzschlußkreislauf integriert ist. Nur damit ist in kurzer Zeit eine nahezu vollständige CO₂ - Umsetzung erreichbar und die Erzeugung eines gut definierbaren Gasproduktes möglich. Die erfindungsgemäßen Vorteile bestehen also darin, dass sowohl das zu verwertende als auch das produzierte Gas den Reaktor zielgerichtet, so oft wie erforderlich, passieren müssen und damit sowohl eine maximale CO₂ - Ausnutzung als auch eine optimale Gasreinigung gewährleistet sind. Das Verfahren ist somit auch zur Entfernung von CO₂ aus der Luft geeignet.

Vorteilhafterweise können dem System über diesen Kreislauf bei Bedarf zusätzliche Komponenten zugeführt werden.

Im Gegensatz zu den oben beschriebenen Verfahren steht bei dem erfindungsgemäßen Verfahren nicht die Produktion von Algenbiomasse im Vordergrund, sondern einer Mischkultur aus Algen und anderen Mikroorganismen zur Gewinnung von Biomasse auf der Basis kohlenstoffhaltiger Gase.

Der erfindungsgemäße Reaktor mit geschlossenem Gaskreislauf ist so konstruiert, dass eine nahezu vollständige Assimilation des bereitgestellten CO₂ erfolgen kann und bei einer Speisung mit Biogas ein eventuell Stickstoffreste enthaltendes Gemisch aus Sauerstoff und Methan den Reaktor verlässt. Dieses Gasgemisch kann unmittelbar energetisch verwertet oder einer Sauerstoffabtrennung zugeführt werden. Nach der Sauerstoffabtrennung liegt ein hochwertiges Methangas vor, das einer vielfältigen Verwendung zugeführt werden kann. Auch der Sauerstoff kann getrennt verwertet werden. Bei einer ausschließlichen Kohlendioxid-Zuführung kann biologisch gebildeter Sauerstoff gewonnen werden. In Verwirklichung der Aufgabenstellung sind verschiedene Ausführungen möglich, die einzeln oder in Kombination angewendet werden können.

Der erfindungsgemäße Photobioreaktor/das erfindungsgemäß Verfahren umfasst die folgenden Merkmale:
- das Strömungssystem wurde gasdicht gestaltet,
- das Reaktorsystem verfügt über einen oder mehrere gesonderte Gaskreisläufe,
- das außerhalb des eigentlichen Reaktors im Kreislauf geführte Gas muss im Ein- und Ausgang bei Bedarf Sterilfilter passieren,
- der Reaktor ist mit einer Mess- und Regelstrecke versehen, welche die Gaseinleitung über entsprechende Ventile steuert,
- nach Erreichen des justierten Gasdargebotes wird die Gaszufuhr unterbrochen,
- das eingeleitete Gas verweilt so lange im Gaskreislauf, bis soviel verwertbares Gas durch die Zellen verbraucht ist, dass erneut Gas eingeleitet wird,
- die phototrophen Zellen können sowohl Mikroorganismen als auch Pflanzenzellen sein, beliebige andere gasverwertende Zellen können eingesetzt werden,
- in das Strömungssystem sind Sensoren zur Überwachung weiterer Gaskomponenten integriert,
- die Beleuchtung erfolgt wahlweise natürlich (Tageslicht) oder künstlich oder kombiniert,
- die Biomassenentnahme aus dem Reaktor wird kontinuierlich oder diskontinuierlich realisiert.

Die Vorteile des Reaktorsystems liegen vor allem darin, dass es sich um ein System mit geschlossenem Gaskreislauf handelt, das eine effektive Verwertung der dargebotenen Nährgase durch die in der Reaktorflüssigkeit befindlichen Zellen ermöglicht. Dabei wird auch der Eintrag von möglichen Verunreinigungen aus der Umgebungsluft vermieden. Damit ist der Reaktor nicht nur für die Produktion von Biomasse, sondern speziell auch für die biotechnologische Produktion verwertbarer Gase geeignet. Des Weiteren ist das Zellmaterial im Vergleich zu einem Röhrenreaktorsystem geringeren mechanischen Belastungen ausgesetzt. Der Reaktor ist sterilisierbar, was in Abhängigkeit von der Verwertung der Biomasse von Bedeutung sein kann.

Die Erfindung soll nachfolgend anhand von Ausführungsbeispielen erläutert werden.
- Figur 1: ist eine schematische Darstellung eines erfindungsgemäßen Systems;
- Figur 2: ist eine schematische Darstellung einer weiteren Ausführungsform eines erfindungsgemäßen Systems;
- Figur 3: stellt eine Prinzipskizze eines als Blasenreaktorsystem ausgestalteten Photobioreaktors mit geschlossenem Gaskreislauf dar.

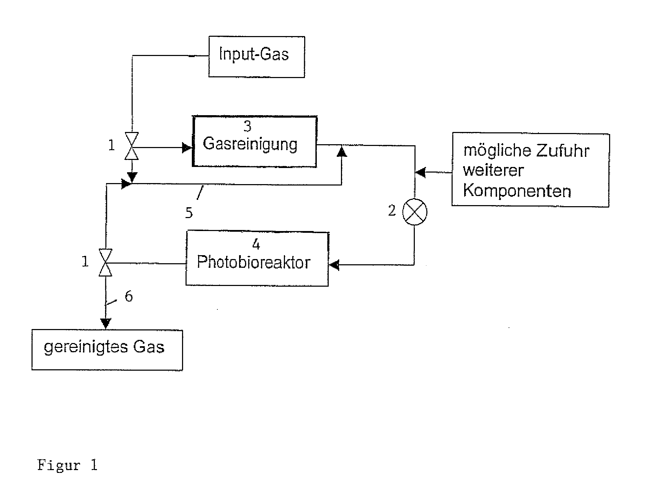

Gemäß Figur 1 wird das Input-Gas über ein sensorgesteuertes Dreiwegeventil 1 zunächst einer Gasreinigung 3 zugeführt. Eine Pumpe 2 führt das gereinigte Input-Gas in einen geschlossenen Photobioreaktor 4. Das den Photobioreaktor 4 verlassende Gas wird je nach CO₂-Entfernungsgrad wiederum über ein sensorgesteuertes Dreiwegeventil 1 in den Gaskreislauf 5 oder als gereinigtes Gas zur weiteren Verwendung durch die Leitung 6 aus dem System geführt. Nicht dargestellte Sensoren zur Überwachung verschiedener Gaskomponenten, wie Methan, Kohlendioxid und Sauerstoff, steuern die Ventile 1. Außerdem können vor der Pumpe 2 weitere Komponenten zugefügt werden. In den Gaskreislauf 5 kann bei Bedarf unter Umgehung der Gasreinigung 3 auch Input-Gas eingespeist werden.

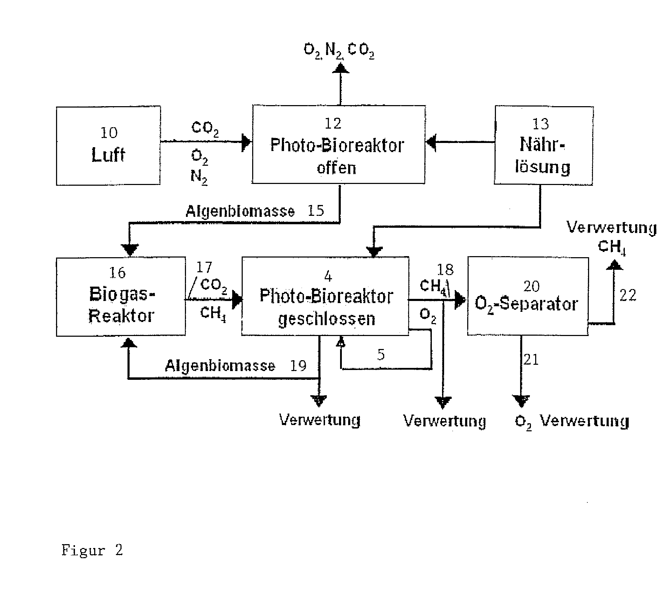

Gemäß Figur 2 dient als Ausgangsmaterial für die Methanproduktion Umgebungsluft 10. Dieselbe wird einem offenen Photobioreaktor 12 zugeführt, in dem mit Hilfe von Licht und zugeführter Nährlösung 13 aus dem Kohlendioxid der Luft Algenbiomasse produziert wird. Das überschüssige Gas, bestehend aus dem nicht umgesetzten Kohlendioxid, Stickstoff und Sauerstoff entweicht in die Umgebung. Die entstandene Biomasse 15 wird einem Anaerobreaktor 16 zugeführt und in demselben in Methan und Kohlendioxid umgewandelt. Das entstandene Biogas 17 wird vorzugsweise nach Passage einer Filtermasse zur Schwefelbeseitigung (nicht dargestellt) einem geschlossenen Photobioreaktor 4 zugeführt und gemäß Ausführungsbeispiel 1 in Sauerstoff und Methan umgesetzt, wobei es über die im Kreislauf geführte Leitung 5 den Photobioreaktor so oft passiert, bis die gewünschte CO₂-Entfernung erreicht ist. Die entsehende Algenbiomasse 19 kann verwertet oder zurück in den Biogasreaktor 16 geführt werden. Das Reingas 18 kann verwertet oder einem Sauerstoffseparator 20 zugeführt werden, wobei die O₂-Verwertung 21 und die CH₄-Verwertung 22 getrennt erfolgt.

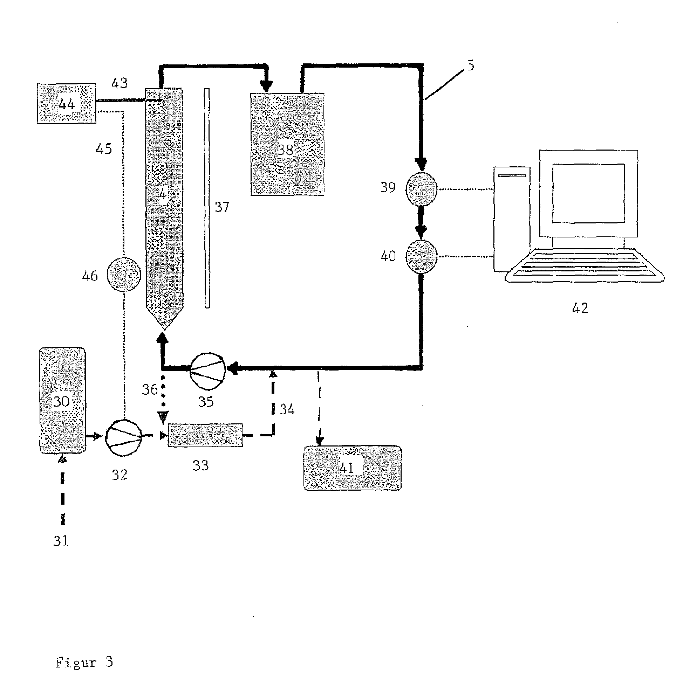

Das in Figur 3 dargestellte Reaktorsystem realisiert einen geschlossenen Gaskreislauf. Kernstück des Reaktorssystems ist ein Photobioreaktor 4 aus Glas. Die Funktionsweise des Reaktorsystems lässt sich wie folgt beschreiben:
Aus einem Biogasspeicher 30 wird das Rohgas durch eine Pumpe 32 zunächst über eine Gasreinigungsmasse 33 geleitet. Dabei wird Schwefelwasserstoff aus dem Rohgas entfernt. Anschließend erfolgt die Einspeisung 34 des Biogases über eine weitere Pumpe 35 in den (geschlossenen) Gaskreislauf 5. Im Photobioreaktor 4 erfolgt die Umsetzung des im Biogas vorhandenen CO₂ infolge der Photosyntheseleistung der Mikroalgen in Biomasse. Die akkumulierte Biomasse wird über eine Bypassvorrichtung 36 an der Kegelspitze am unteren Ende des Photobioreaktors 4 entnommen. Die Beleuchtung des Photobioreaktors 4 erfolgt mit einer Leuchtstoffröhre 37. Das aus dem Photobioreaktor 4 ausgetragene Gas passiert zunächst eine Kühlfalle 38 zur Kondensation von mitgeschleppter Flüssigkeit. Anschließend durchläuft das gereinigte Gas eine Sensoreinheit 39, 40 zur Überwachung der aktuellen CH₄- und CO₂-Gehalte. Die Daten werden von einem PC 42 online erfasst. Das aufbereitete Gas verlässt nach den erforderlichen Zirkulationszyklen den geschlossenen Gaskreislauf 5 und wird in einem Gasspeicher 41 gesammelt.

Das Reaktorsystem ist so konstruiert, dass das eingeleitete Biogas mindestens einmal den gesamten Kreislauf und damit auch den Reaktor durchlaufen hat, bevor es aufgrund des Überdrucks wieder ausgeleitet wird. Dazu ist das Reaktorsystem mit einer Mess- und Regelstrecke 43, 44, 45 versehen. Übersteigt der mittels pH-Elektrode 43 in der Mikroalgensuspension gemessene pH-Wert einen vorgegebenen Wert, aktiviert das Steuerelement 44 die Pumpe 32 und Biogas wird in den Reaktor 4 eingeleitet. Nach Erreichen des justierten pH-Wert-Minimums wird die Gaszufuhr abgebrochen. Das eingeleitete Gas verweilt dann so lange im System, bis durch den Verbrauch des CO₂ durch die Algen der pH-Wert wieder ansteigt und erneut Gas eingeleitet wird. Ein Zähler 46 registriert die Dauer der Einleitung von Biogas und ermittelt damit gleichzeitig die Produktion des Zielgases, d h. einem Gasgemisch, dass vorwiegend aus Methan und Sauerstoff besteht. Wahlweise kann in den Gaskreislauf ein Sauerstoffseparator integriert werden. Eine Zuleitung 31 aus einem Biogasreaktor kann optional ebenfalls vorgesehen sein.

## Patentansprüche

1. Verfahren zur Verwertung gasförmiger Kohlenstoffquellen, **dadurch gekennzeichnet, dass** das Input-Gas einen gasdicht geschlossenen Photobioreaktor mittels eines geschlossenen Gaskreislaufes so oft durchläuft, bis eine nahezu vollständige biotechnologische Umwandlung von C0₂ in ein Methan-Sauerstoff-Gemisch bzw. in Methan erfolgt ist.

2. Verfahren nach Anspruch 1, **dadurch gekennzeichnet, dass** das Input-Gas so lange im Gaskreislauf verweilt, bis soviel gasförmige Nährstoffe durch die in der Flüssigphase enthaltenen Zellen verbraucht sind, dass erneut Gas eingeleitet wird.

3. Verfahren nach Anspruch 1 oder 2, **dadurch gekennzeichnet, dass** das im Kreislauf geführte Gas vollständig von der Berührung mit Umgebungsluft bzw. anderen unerwünschten Gaskomponenten abgeschirmt ist, bis es das Reaktorsystem zweckbestimmt verlässt.

4. Verfahren nach Anspruch 1, 2 oder 3, **dadurch gekennzeichnet, dass** das Input-Gas vor dem Einleiten in den Photobioreaktor gereinigt wird.

5. Verfahren nach einem der vorhergehenden Ansprüche, **dadurch gekennzeichnet, dass** das außerhalb des eigentlichen Reaktors im Kreislauf geführte Gas im Ein- und Ausgang des Reaktors bei Bedarf Sterilfilter passieren muss.

6. Verfahren nach einem der vorhergehenden Ansprüche, **dadurch gekennzeichnet, dass** dem Kreislauf bei Bedarf zusätzliche Komponenten zugeführt werden.

7. Verfahren nach einem der vorhergehenden Ansprüche, **dadurch gekennzeichnet, dass** dem Gaskreislauf unter Umgehung der Gasreinigung Input-Gas zugeführt werden kann.

8. Verfahren nach einem der vorhergehenden Ansprüche, **dadurch gekennzeichnet, dass** Kohlendioxid rein oder in einem Gasgemisch enthalten einem offenen Photobioreaktor zugeführt, dort mittels Photosynthese in Biomasse umgewandelt, überschüssiges Gas in die Umgebung abgegeben und die gebildete Biomasse einem Biogasreaktor zugeführt wird und das in diesem entstehende Biogas unmittelbar in den gasdicht geschlossenen Photobioreaktor übergeleitet und im geschlossenen Gaskreislauf geführt wird.

9. Verfahren nach Anspruch 8, **dadurch gekennzeichnet, dass** das im Gaskreislauf des geschlossenen Bioreaktors geführte Gas seine verwertbaren Kohlenstoffanteile nahezu vollständig an die flüssige Reaktorphase abgibt und die durch die Zellen der Flüssigphase produzierten Gaskomponenten nahezu vollständig über den geschlossenen Gaskreislauf abgeführt werden sowie die gebildete Biomasse wiederum dem Biogasreaktor zugeführt wird.

10. Verfahren nach einem oder mehreren der vorhergehenden Ansprüche, **dadurch gekennzeichnet, dass** das gebildete Gasgemisch direkt energetisch verwertet oder einer Sauerstoffabtrennung zugeführt wird.

11. Verfahren nach einem oder mehreren der vorherigen Ansprüche, **dadurch gekennzeichnet, dass** nach der Sauerstoffabtrennung ein hochwertiges Methangas aus dem System ausgeführt und einer vielfältigen Verwendung zugeführt wird.

12. Verfahren nach einem oder mehreren der vorherigen Ansprüche, **dadurch gekennzeichnet, dass** die gebildete Biomasse einer gesonderten Verwertung zugeführt wird.

13. Verfahren nach einem oder mehreren der Ansprüche 8 bis 12, **dadurch gekennzeichnet, dass** dem Biogasreaktor zusätzliche Biomasse zugeführt wird.

14. Verfahren nach einem oder mehreren der vorhergehenden Ansprüche, **dadurch gekennzeichnet, dass** die Gaseinleitung über sensorgesteuerte Ventile geregelt wird.

15. Verfahren nach einem oder mehreren der vorhergehenden Ansprüche, **dadurch gekennzeichnet, dass** die Gasführung mittels einer Mess- und Regelstrecke gesteuert wird.

16. Verfahren nach einem oder mehreren der vorherigen Ansprüche **dadurch gekennzeichnet, dass** die Stoffzugaben und - entnahmen aus dem Reaktor kontinuierlich oder diskontinuierlich realisiert werden.

17. Photobioreaktor zur Verwertung gasförmiger Kohlenstoffquellen, **dadurch gekennzeichnet, dass** derselbe gasdicht ausgeführt ist und über einen geschlossenen Gaskreislauf verfügt.

18. Photobioreaktor nach Anspruch 17, **dadurch gekennzeichnet, dass** in das Strömungssystem Sensoren zur Überwachung verschiedener Gaskomponenten, z. B. Methan, Kohlendioxid und Sauerstoff integriert sind.

19. Photobioreaktor nach Anspruch 17 oder 18, **dadurch gekennzeichnet, dass** der Reaktor mit einer Mess- und Regelstrecke versehen ist, welche bei Bedarf die Gaseinleitung über entsprechende Ventile steuert.

20. Photobioreaktor nach einem der Ansprüche 17 bis 19, **dadurch gekennzeichnet, dass** die phototrophen Zellen sowohl Mikroorganismen als auch Pflanzenzellen sein können.

21. Photobioreaktor nach einem der Ansprüche 17 bis 20, **dadurch gekennzeichnet, dass** die Beleuchtung wahlweise natürlich (Tageslicht) oder künstlich oder kombiniert erfolgt.
